# EUROPEAN PATENT APPLICATION

(11) **EP 4 710 962 A1**
(43) Date of publication of application: **18.03.2026**
(21) Application number: 24200574.2
(22) Date of filing: 16.09.2024
(51) Int. Cl.: A61L 29/04, A61L 29/08, A61L 29/14

(54) **TIE-LAYER COATING FOR CATHETER LINERS**

(71) Applicant: Zeus Company LLC, Orangeburg SC 29118 (US)
(72) Inventor: SINGHI, Bhavya, Orangeburg, South Carolina 29115 (US); CAMPANELLI, John Richard, Orangeburg, South Carolina 29115 (US); YOUNAN, Alexandra, Orangeburg, South Carolina 29115 (US); COOPER, Patrick, Orangeburg, South Carolina 29115 (US); BALLARD, Robert. L., Orangeburg, South Carolina 29115 (US); RAST III, Edward H., Orangeburg, South Carolina 29115 (US); MARRO, Justin, Orangeburg, South Carolina 29115 (US); BARRINGER, Dana, Orangeburg, South Carolina 29115 (US)
(74) Representative: Hofstetter, Schurack & Partner

(57) **Abstract**

The disclosure provides coated tubings, including a layer of PEBA (or other polymer) on an etched polymeric tubing. The layer of PEBA (or other polymer) can have a low thickness, e.g., less than 1 micron and can provide for efficient bonding to an overlying jacket layer. The disclosure further provides methods of making and using such coated tubings.

## Description

### FIELD OF THE INVENTION

The present application is directed to functionalized plastic tubings and methods for making such functionalized plastic tubings, which find application in a variety of fields.

### BACKGROUND

Polytetrafluoroethylene (PTFE) tubular liners are known in the art and widely used, e.g., in catheter manufacturing. In the context of catheter manufacturing, the PTFE liner is commonly used as the first (innermost) layer of a catheter. PTFE has been an ideal material for inner liners of catheters due to the chemical resistance, biocompatibility, and low coefficient of friction (COF) of PTFE. PTFE exhibits unique characteristics in this field that other polymers do not exceed. With such a low COF, PTFE has been able to provide an inner diameter that easily allows various catheter technologies such as stents, balloons, atherectomy or thrombectomy devices to be pushed through a small diameter catheter lumen. The effect of increased lubricity of the catheter inner diameter (ID) is a reduced deployment force of catheter devices as the catheter devices are passed through the lumen of the catheter ID, increasing the likelihood of a successful procedure.

To promote adhesion between the outer surface of the PTFE liner and an adjacent outer/jacket layer, e.g., polyether block amide (PEBA), the outer surface of the PTFE liner is typically etched. A well-known drawback of the etching process used to functionalize the surface of PTFE liners is that the etched surface changes over time as it adsorbs reactive species from its surroundings. As a result of this reactive adsorption process, the prevalence of bonding sites on the PTFE liner for adhesion to the jacket layer decreases as the surface ages. Known technologies make use of phenol-based coatings for applying PEBA jacket layers. A similar product made by casting is taught by: L. Liu, A. Chakman, X. Feng, "A Novel Method of Preparing Ultrathin Poly(Ether Block Amide) Membranes," J. Membrane Sci., 235, 43-52, 2004. Liu *et al.* use n-butanol/isopropanol mixtures to dissolve a very soft PEBA, Altofina 2533, in order to cast membranes. They report no tests with harder grades of PEBA or applications with coating of tubes.

It would be useful to provide alternative tubular liners capable of sufficient bonding to overlying jacket layers in catheter construction and methods of providing such tubular liners.

### SUMMARY

The disclosure generally provides tubings comprising a thin layer of tie-resin (also referred to herein as a "tie layer") such as PEBA, nylon, polyurethanes, and/or polyesters of various hardnesses coated onto functionalized plastic tubing. The functionalized plastic tubing can be, *e.g.,* an etched PTFE liner, or plasma treated polyethylene, which can be used in catheter shaft constructions. The tie-resin layer bonds to the functionalized outer surface of the functionalized plastic tubing and provides adhesion to other materials applied thereover, including PEBA, nylon, polyurethane, etc. tubes that are subsequently reflowed over the liner/tie resin and braid assembly during catheter construction.

Coating the etched surface with a thin tie-resin layer as provided herein can act to preserve adhesion by preventing further aging of the surface of the underlying functionalized plastic tubing. If the tie-resin layer is applied in a secondary step, some surface aging will nevertheless occur prior to that coating step. In some embodiments, the disclosed tie layer application process can be performed in-line immediately following the surface etching operation performed on the functionalized plastic tubing, thereby minimizing/eliminating the aging effect of the etched surface.

The disclosure includes, without limitations, the following embodiments.
Embodiment 1: A coated tubing, comprising an etched PTFE tubing with an inner surface and an outer surface, wherein the outer surface comprises a layer of PEBA thereon with an average thickness of less than 1 micron.
Embodiment 2: The coated tubing of Embodiment 1, where the PEBA has a durometer of 25 to 72.
Embodiment 3: The coated tubing Embodiment 1 or 2, prepared using a PEBA-containing solution.
Embodiment 4: The coated tubing of Embodiment 3, wherein the PEBA-containing solution comprises a solvent selected from methanol, ethanol, 2-methyl-2-propanol, 1-butanol, 2-butanol, and mixtures thereof.
Embodiment 5: The coated tubing of Embodiment 3, where the PEBA-containing solution comprises 1-butanol.
Embodiment 6: The coated tubing of any of Embodiments 3-5, wherein the PEBA is present in the PEBA-containing solution at a wt/vol ratio of less than 0.10 g/mL.
Embodiment 7: The coated tubing of any of Embodiments 3-6, wherein the PEBA-containing solution is at a temperature of 20°C to 100°C at the time of coating.
Embodiment 8: The coated tubing of any of Embodiments 1-7, wherein the average thickness of the coating is below 0.9 µm.
Embodiment 9: The coated tubing of Embodiment 8, where the average thickness of the coating is below 0.8 µm.
Embodiment 10: The coated tubing of any of Embodiments 1-9, further comprising a jacket layer comprising PEBA or a polyamide coated on the layer of PEBA.
Embodiment 11: The coated tubing of any of Embodiments 1-10, wherein the layer of PEBA consists essentially of PEBA.
Embodiment 12: The coated tubing of any of Embodiments 1-10, wherein the layer of PEBA comprises one or more additives.
Embodiment 13: The coated tubing of any of Embodiments 1-12, wherein the layer of PEBA is substantially free of residual solvent.
Embodiment 14: The coated tubing of any of Embodiments 1-13, wherein the coated tubing is a catheter liner.
Embodiment 15: A catheter comprising the coated tubing of any of Embodiments 1-14.
Embodiment 16: A coated tubing comprising a tubing comprising a fluoropolymer, with an inner surface and an outer surface, wherein the outer surface comprises a tie-layer thereon, wherein the tie-layer has a thickness of less than 1 micron.
Embodiment 17: A method of preparing a coated tubing with an inner surface and an outer surface, comprising: providing an etched PTFE tubing with an inner surface and an outer surface; dissolving PEBA in one or more solvents to provide a PEBA-containing solution; and applying the PEBA-containing solution to the outer surface of the etched PTFE tubing to give a layer of PEBA thereon with an average thickness of less than 1 micron.
Embodiment 18: The method of Embodiment 17, wherein the dissolving comprises heating and/or agitating the PEBA in one or more solvents.
Embodiment 19: The method of Embodiment 17 or 18, wherein the PEBA-containing solution comprises a solvent selected from non-aromatic alcohols.
Embodiment 20: The method of Embodiment 19, wherein the non-aromatic alcohol is selected from methanol, ethanol, 2-methyl-2-propanol, 1-butanol, 2-butanol, and mixtures thereof.
Embodiment 20: The method of any of Embodiments 17-20, wherein the PEBA-containing solution comprises a solvent selected from glycols, diols, and ketones.
Embodiment 21: The method of any of Embodiments 17-20, wherein the PEBA-containing solution does not comprise an aromatic alcohol.
Embodiment 22: The method of any of Embodiments 17-21, wherein the applying comprises physically coating, chemically coating, dip-coating or spraying the PEBA-containing solution onto the etched PTFE tubing.

These and other features, aspects, and advantages of the disclosure will be apparent from a reading of the following detailed description together with the accompanying drawings, which are briefly described below. The invention includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosed invention, in any of its various aspects and embodiments, should be viewed as intended to be combinable unless the context clearly dictates otherwise.

### BRIEF DESCRIPTION OF THE DRAWINGS

In order to provide an understanding of the embodiments of the invention, reference is made to the appended drawings, which are not necessarily drawn to scale, and in which reference numerals refer to components of exemplary embodiments of the invention. The drawings are exemplary only, and should not be construed as limiting the invention.
FIG. 1 is a general schematic of a functionalized plastic tubing **10,** which is a component of the tubings of the present disclosure, with relevant parameters, and an expanded schematic of one cross-sectional end face of the tubing;
FIG. 2 is a general schematic of a tubing **15** provided according to certain embodiments of the disclosure, comprising functionalized plastic tubing **10** and tie layer coating **12** thereon.

### DETAILED DESCRIPTION

The present invention will now be described more fully hereinafter with reference to the accompanying figures, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

The present disclosure provides functionalized plastic tubings comprising a tie-layer coating on an outer surface thereof, which tie-layer coating is in direct contact with an outer polymeric layer. The disclosure further provides methods of preparing and using such assemblies.

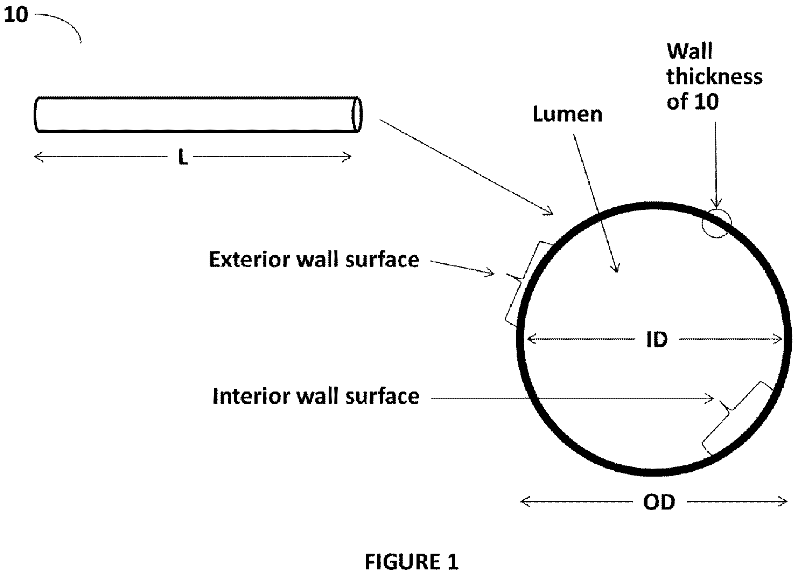

The underlying functionalized plastic tubing referenced herein can be better understood by reference to FIG. 1, wherein functionalized plastic tubing **10** is depicted in longitudinal and cross-sectional form. The size of the plastic tubing (e.g., inner diameter (ID), outer diameter (OD), wall thickness, length L, etc.) is not particularly limited, as the disclosure is broadly applicable to a range of plastic tubings. In some embodiments, the plastic tubing is a thin-walled tubing, although the disclosure is not limited thereto. In some embodiments, the plastic tubing can have an average wall thickness of 0.1 mm or less, e.g., about 0.09 mm or less, about 0.08 mm or less, about 0.07 mm or less, about 0.06 mm or less, or about 0.05 mm or less, such as about 0.01 mm to about 0.1 mm. The ID (which determines the diameter of the lumen) can vary and, in some embodiments, is of a size suitable for use in catheter applications, e.g., as a liner. The outer diameter of the tube, shown as "OD," is the average distance from a point on the outer wall of the tube through the lumen of the tube to the opposite/farthest point on the outer wall of the tube. As such, half of the OD value minus the ID value provides the average wall thickness of the tube.

The composition of the functionalized plastic tubing is not particularly limited; in preferred embodiments, the plastic tubing may comprise any material suitable for use as an inner layer/liner of a catheter assembly. Such materials include, but are not limited to, fluoropolymers such as poly(tetrafluoroethylene) (PTFE), fluorinated ethylene propylene (FEP), polyfluoroalkoxy (PFA), polyvinylidene fluoride (PVDF), and derivatives, copolymers, and mixtures thereof. In some embodiments, the functionalized plastic tubing may consist essentially of the referenced polymer(s). In other embodiments, the functionalized plastic tubing can comprise one or more additives, which may be intentionally added and/or may be remnants of the resin(s) used to produce the tube (e.g., lubricants or other processing additives).

By "functionalized" is meant that the OD of the plastic tubing is processed in some way, e.g., to promote adhesion between the OD surface and a material to be applied thereto. In some embodiments, the functionalization comprises etching. Methods of etching such plastic tubings and commercially available etched tubings are known. In some embodiments, the etching is conducted as described in further detail herein below, e.g., in line with the plastic tubing production. Other surface functionalization methods known to enhance bonding between two plastic surfaces (e.g., by activating the surface of the plastic tubing to improve bonding characteristics) can also be employed in various embodiments.

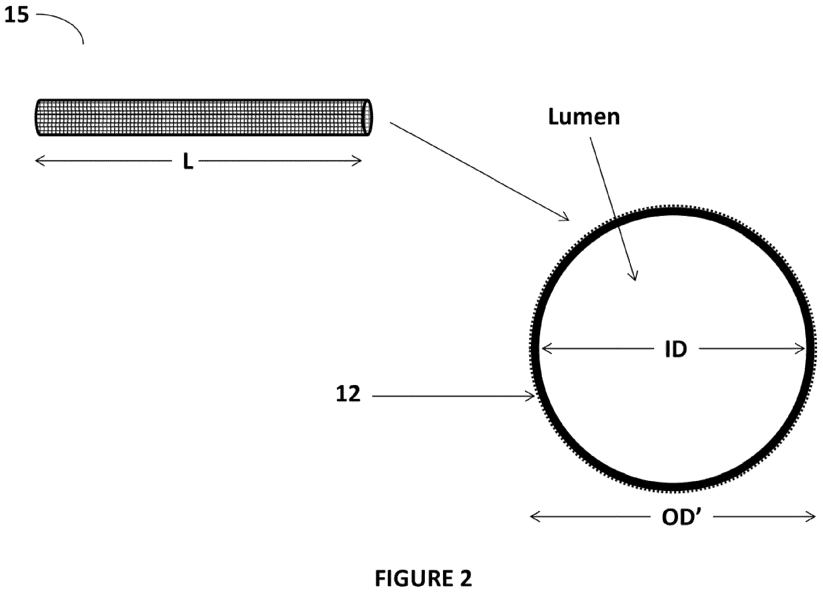

FIG. 2 illustrates a tubing **15** comprising the functionalized plastic tubing **10** with a tie layer **12** overlying and in direct contact with the surface of the OD of the tubing **10.** A "tie layer" (**12**) as used herein is intended to mean a thin layer of a polymeric material. In some embodiments, the tie layer has an average thickness of about 10 microns or less, about 5 microns or less, about 4 microns or less, about 3 microns or less, about 2 microns or less, about 1 micron or less, about 0.9 micron or less, or about 0.8 micron or less, e.g., about 0.1 micron to about 10 microns, about 0.5 micron to about 10 microns, about 0.1 micron to about 5 microns, about 0.5 micron to about 5 microns, about 0.8 micron to about 10 microns, about 0.8 micron to about 5 microns, about 0.1 micron to about 1 micron, about 0.1 micron to about 0.8 micron, or about 0.1 to about 0.5 micron. As such, the OD will be slightly increased relative to the functionalized plastic tubing alone (and thus indicated as OD' in FIG. 2). In certain embodiments, the thickness is substantially uniform, i.e., it does not vary significantly along the length of the tubing or around the circumference of the tubing. For example, for a 1 micron average thickness tie layer, the deviation may be less than 0.1 micron, less than 0.05 micron, less than 0.01 micron, less than 0.005 micron, or less than 0.001 micron. It is noted that the tie-layer **12** is preferably substantially uniform in application as well, e.g., comprising little to no pinholes in the coating. Other types of coatings (prepared by other methods) may not exhibit such high levels of uniformity.

The composition of the tie layer is not particularly limited. In certain embodiments, the tie layer comprises a polyether block amide (PEBA). Various grades of PEBA are known and can be used according to the present disclosure. In some embodiments, the tubes provided herein can be prepared with a wide range of PEBA grades, e.g., PEBAs of varying durometers. For example, in some embodiments, the tie layer can comprise PEBA with a shore hardness as high as 72D to very soft grades, such as 25D. In some embodiments, the shore hardness of the PEBA is about 50D to about 72D and in some embodiments, the shore hardness of the PEBA is about 25D to about 50D. In some embodiments, the tie layer can comprise one or more nylons, polyesters, polyethers, and/or copolymers, derivatives, and/or mixtures thereof.

In some embodiments, the tie layer consists essentially of the polymeric material (e.g., PEBA). For example, in certain embodiments, the tie layer comprises no anti-block or slip additives. In some embodiments, the tie layer comprises a single polymeric material and in other embodiments, the tie layer can comprise a mixture of one or more polymeric materials (e.g., multiple PEBA resins). In some embodiments, the tie layer may comprise one or more additives, e.g., including, but not limited to, lubricants or processing aids which may remain from the resin used to prepare the layer. In preferred embodiments, the tie layer comprises substantially no solvent (i.e., little to no detectable solvent), e.g., less than about 10% by weight, less than about 5% by weight, less than about 1% by weight, less than about 0.5% by weight, less than about 0.1% by weight, or less than about 0.05% by weight of a solvent, based on the total weight of the tie layer.

In preferred embodiments, this minimal solvent content provides for a tie layer that can be described as not tacky. Tackiness can be quantified, for example, by using a DMA to calculate the quantity tan (δ)/E' at low frequency for the coating formulation in question (from: N. Akram, M. Saeed, M. Usman, Polymers, 14, p. 572, 2022). The greater its value, the higher the tackiness of the polymer. Tackiness of the material can also be measured by mechanically bonding two tubing pieces of the coated material, at room temperature under a constant pressure for known period of time and measuring the force for separating the samples using an Instron mechanical tester. Additionally, other surface analyzing instruments/methods such as AFM and SEM may be used to detect and/or measure tackiness of materials.

Polymers such as PEBA, nylons, polyesters, polyethers and their copolymers are subject to attack by alcohols, especially at high temperatures and long duration times in solution. The tubing provided herein exhibits a lower tendency to alcoholysis because the solution dries faster at lower temperatures than commercially available solutions.

Tubings according to various embodiments as provided herein have several advantages over known tubes, including, but not limited to, the following features:
- Lower tackiness of the cast tie layer for a given hardness. Any residual solvent, say from high boiling-point solvent systems, tends to increase the tackiness of the tie layer. Various embodiments are cast from a solvent with a low boiling point that leaves no residual solvent after drying in-line.
- Can accommodate low durometer PEBA layers (e.g., 25 to 72, e.g., 35D and 25D).
- Higher peel strength values after reflow.
- Can be stretched more without loss of adhesion.
- Thinner applied coating with wider range of coating thickness. Coating thicknesses such as 0.5 µm or lower can be achieved.

The disclosure also provides methods of preparing tubings as described herein. In certain embodiments, the tie layer is applied to the functionalized plastic tubing by providing the tie-layer material in liquid form (e.g., in the form of a solution, suspension, dispersion, or the like) and applying the solution to the functionalized plastic tubing. In preferred embodiments, the tie-layer material is provided in the form of a solution. The solution, suspension, dispersion, of the like can be formed by combining the tie layer material (e.g., resin) and one or more solvents. The solvent(s) can vary. In certain embodiments, the solvent(s) can comprise single component or multicomponent mixtures of solvents such as non-aromatic alcohols (e.g., including, but not limited to, methanol, ethanol, 2-methyl-2-propanol, 1-butanol, and 2-butanol), glycols, diols, ketones, and the like. In certain embodiments, the solvent does not comprise an aromatic component, e.g., does not comprise an aromatic alcohol. The solution can be modified to give a wide range of target tie-layer thicknesses on the functionalized polymer tubing (e.g., etched PTFE tube). Methods of adjusting the target thickness include: modifying nominal concentration of polymer in the solution, solution viscosity during processing, application temperature, line speed, drying rate, sizing die gap, and number of coating passes (e.g., one versus multiple).

The concentration of the tie-layer material within the solutions provided herein can vary and, accordingly, the viscosity of the solutions can vary. In some embodiments, the tie-layer material is present in the solution at a wt/volume ratio of less than 0.10, e.g., about 0.02 to about 0.10. Weight/volume ratios as provided herein are provided in grams of polymer per milliliter of solvent, unless otherwise expressly indicated. In some embodiments, the viscosity of the solution can be 10 to 200 cP, e.g., at 60°C (and can be within this range or higher at temperatures below 60°C/within this range or lower at temperatures above 60°C). The tie-layer solutions used in the current invention have a significant temperature-viscosity relationship. Altering the solution temperature can dramatically affect the viscosity and as such, one may modify the temperature of solvation/application to the functionalized plastic tubing to modify the viscosity of the solution. For example, a specific concentration of 55D Pebax solution has a much higher viscosity at 60 °C than it does at 80 °C.

Pebax polymers are thermoplastic elastomers (TPE) that consists of block copolymers made up of polyamide and polyether blocks. By adjusting the composition and ratio of these blocks, a wide range of flexibility can be achieved, from stiff and rigid to soft and flexible, without the use of plasticizers. PEBA is a more general term for this category of polymers.

These distinct polymers possess the advantageous characteristics of both polyamides and polyethers/polyesters. They have the strength and durability associated with polyamides, along with the flexibility and elasticity that are typical of polyethers and polyesters.

In one embodiment, a 10% (wt/vol) 55D Pebax solution in 1-butanol had a viscosity of less than 50cP at 80°C, while a 5% (wt/vol) 55D Pebax solution in 1-butanol at 50°C had a viscosity of greater than 150cP. The wt/vol ratio is defined as grams of PEBA (or other polymeric material) divided by milliliters of solvent. According to the present disclosure, various temperatures can be employed for solvation of the tie-layer material and/or the application of the tie-layer material and as such, a wide range of solution viscosities can be used.

The grades of tie-resins that can be accommodated with these solvent systems range from a Shore hardness as high as 72D to very soft grades, such as 25D. The conditions for solvation of the resin(s) can vary, and may optionally involve heating and/or agitation. Solvation for the harder grades can be accomplished, e.g., at temperatures above ambient in a closed container with agitation (e.g., within a few hours).

Applying can be by various means, including, but not limited to, physical coating, chemical coating, dip-coating or spraying the tie-layer material in liquid form (e.g., the tie layer solution) to the functionalized plastic tubing, e.g., via conventional methods known in the art. The solvent of the tie-layer solution applied to the functionalized polymer tubing (e.g., comprising an alcohol) is generally easily removed after application of the solution to the functionalized polymer tubing by drying with, for example, ambient air, heated air, convection, infrared heat, or radiant heat. The conditions for such drying (e.g., temperature) can be selected based, e.g., on the solvent used in the application process. Additionally, the coated tubes may be quenched with water or cold air before going through a drying step.

Various embodiments provide stable processing for manufacturing the tubings as descried herein. The pot life at processing temperatures is adequate for continuous application to a functionalized polymer tubing (e.g., an etched PTFE tube), e.g., over an eight-hour shift).

The process used to make tubings according to various embodiments provided herein also has several advantages over conventional processes:
- An Environmental Health and Safety (EH&S)-friendly solvent system can be used for applying the tie layer coating (versus current aromatic alcohols and cosolvent system).
- Faster processability (higher throughputs can be achieved due to faster drying rate).
- The process can be designed to yield continuous lengths of coated PTFE liners.
- Little to no detectable residual solvent in the dried tie layer.

Relative to known tubes (e.g., tubes comprising the same materials but prepared according to different methods), tubes according to various embodiments may exhibit:
- Higher peel strength for reflowed catheter shafts.
- Higher stretching without corresponding loss in peel strength.
- Lower tackiness for soft grades of Pebax (e.g., 25D and 35D) without the need for anti-block or slip additives. Lower tackiness implies easier handling during processing and packaging, as well as easier handling at the customer end (e.g., in connection with use of the tube in catheter construction).
- Thinner coatings (e.g., less than one micron) for improved flexibility. Some embodiments provide coatings with thicknesses of below one micron, below 0.9 µm, or below 0.8 µm.
- Softer grades of Pebax can be used for improved flexibility.
- Adhesive failure mechanism implies complete miscibility with the reflowed Pebax, and consequently lower risk of delamination in the finished catheter.
- Competitive products are made via manual batch process whereas the current invention describes an in-line continuous process.
- Uses a more EH&S-friendly solvent system.
- Tubes according to various embodiments are dried at lower temperature and for a shorter time, which greatly reduces the possibility of Pebax alcoholysis compared to the related art processes which generally require drying at high temperatures (>120°C) for longer times (> 1 min).

### EXAMPLES

Examples of known tie-layer coated tubes, tie-layer coated tubes according to various embodiments of the present disclosure, and uncoated etched PTFE tubes were tested to assess the physical, mechanical and thermal properties including tensile and burst tests, FTIR, DSC, DMA, Contact Angle, NMR, HPLC, GPC, tackiness, coating thickness, coating uniformity (e.g., concentricity).

### Example 1:

5% (wt./vol) solution of Pebax 5533 and 1-butanol was prepared using a heated water bath at 90°C. The solution was used to dip-coat etched PTFE tubing with an OD of 0.081" at a temperature of 70°C in a continuous in-line process. The coated tubing was passed through an oven at 120 °C. The mean coating thickness was determined to be around 0.5 micron by microscopy.

### Example 2:

5% (wt./vol) solution of Pebax 3533 and 1-butanol was prepared using a heated water bath at 90°C. The solution was used to dip-coat etched PTFE tubing with an OD of 0.081" at ambient temperature in a continuous in-line process. The coated tubing was passed through an oven at 120 °C. The mean coating thickness was determined to be around 0.5 micron by microscopy.

### Example 3:

10% (wt/vol) solution of Pebax 5533 and 1-butanol was prepared using a heated water bath at 90°C. The solution was used to dip-coat etched PTFE tubing with an OD of 0.081" at a temperature of 80°C in a continuous in-line process. The coated tubing was passed through an oven at 120 °C. The mean coating thickness was determined to be around 2 microns by microscopy.

### Example 4:

20% (wt/vol) solution of Pebax 2533 and 1-butanol was prepared using a heated water bath at 90°C. The solution was used to dip-coat etched PTFE tubing with an OD of 0.081" at ambient temperature in a continuous in-line process. The coated tubing was passed through an oven at 120 °C. The mean coating thickness was determined to be around 4.5 microns by microscopy.

### Comparative Example 1:

A commercially available Pebax-coated tube obtained from a manual dipping/drying process using an aromatic/hydrocarbon solvent blend was obtained for comparison. The mean coating thickness was determined to be around 1 micron by microscopy.

### Comparative Example 2:

A commercially available Pebax-coated tube obtained from a manual dipping/drying process using an aromatic/hydrocarbon solvent blend was obtained for comparison. The mean coating thickness was determined to be around 2 microns by microscopy.

Many modifications and other embodiments of the invention will come to mind to one skilled in the art to which this invention pertains having the benefit of the teachings presented in the foregoing description. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A coated tubing, comprising an etched PTFE tubing with an inner surface and an outer surface, wherein the outer surface comprises a layer of PEBA thereon with an average thickness of less than 1 micron.

2. The coated tubing of claim 1, where the PEBA has a durometer of 25 to 72.

3. The coated tubing of claim 1 or 2, prepared using a PEBA-containing solution.

4. The coated tubing of claim 3, wherein the PEBA is present in the PEBA-containing solution at a wt/vol ratio of less than 0.10 g/mL.

5. The coated tubing of claim 3 or 4, wherein the PEBA-containing solution is at a temperature of 20°C to 100°C at the time of coating.

6. The coated tubing of any of claims 1 to 5, wherein the average thickness of the layer of PEBA is below 0.9 µm, in particular below 0.8 µm.

7. The coated tubing of any of claims 1 to 6, further comprising a jacket layer comprising PEBA or a polyamide coated on the layer of PEBA.

8. The coated tubing of any of claims 1 to 7, wherein the layer of PEBA consists essentially of PEBA and/or wherein the layer of PEBA comprises one or more additives and/or wherein the layer of PEBA is substantially free of residual solvent.

9. The coated tubing of any of claims 1 to 8, wherein the coated tubing is a catheter liner.

10. A catheter comprising the coated tubing of any of claims 1 to 9.

11. A method of preparing a coated tubing with an inner surface and an outer surface, comprising:
providing an etched PTFE tubing with an inner surface and an outer surface;
dissolving PEBA in one or more solvents to provide a PEBA-containing solution; and
applying the PEBA-containing solution to the outer surface of the etched PTFE tubing to give a layer of PEBA thereon with an average thickness of less than 1 micron.

12. The method of claim 11, wherein the dissolving comprises heating and/or agitating the PEBA in one or more solvents.

13. The method of claim 11 or 12, wherein the PEBA-containing solution comprises a solvent selected from non-aromatic alcohols.

14. The method of claim 13, wherein the non-aromatic alcohol is selected from methanol, ethanol, 2-methyl-2-propanol, 1-butanol, 2-butanol, and mixtures thereof.
